# EUROPEAN PATENT APPLICATION

(11) **EP 1 570 731 A1**
(43) Date of publication of application: **07.09.2005**
(21) Application number: 04005271.4
(22) Date of filing: 05.03.2004
(51) Int. Cl.: A01K 23/00, A61F 5/44, A61L 24/00

(54) **Disposable pouch for collecting feces from animals**

(71) Applicant: The Procter & Gamble, Ohio 45202 (US)
(72) Inventor: Cinelli, Fabio, 65124 Pescara (IT); Bonvissuto, Irene, 66020 Sambuceto (Chieti) (IT)
(74) Representative: Veronese, Pancrazio

(57) **Abstract**

A disposable animal or pet fecal management device in the form of a pouch (10) which can be attached directly to the animal body in correspondence with the animal anus, via a body adhesive in the form of a gel. Such a device is easily applied, removed, disposed of, does not leave residues upon removal and is surprisingly well tolerated by animals, particularly dogs.

## Description

### FIELD OF THE INVENTION

This invention relates to a disposable pouch for collecting feces from an animal or pet which is attached directly to the body of the animal in correspondence of the anus using a gel adhesive.

The problem of taking care of animal feces is very important especially in the urban areas where pets are commonly considered as a source of pollution of public places such as streets, gardens and the like.

The problem is particularly relevant for dogs due to high number of families keeping pet dogs. Many devices are currently marketed for removal of dog waste from urban sidewalks, parks etc. and for provision of a hygienic disposal. Most devices of this type, such as for example Skoop-n-pack from Pet Zone ® rely on the dog's owner to collect the feces of the dog from the ground by means of a plastic bag and a scoop which can be plastic (reusable) or carton (for single use). These methods are a nuisance and tend to be embarrassing to dog owners who have to pick up the waste by hand (i.e. feel feces trough the plastic bag/paper), or with a cumbersome poop scoop. Moreover, devices are inconvenient to carry around and therefore often are not available at the right time and/or location. Also, the dog owner is obliged to track all movements of his dog in order to not miss a needed cleanup intervention.

Other devices addressing the problem with a different approach are described in the art such as those described in US 4,813,949, US 5,555,847, US 4,996,949, US 6,368,313. These devices resemble human diapers which are kept in place with belts, adhesive stripes and the like. These articles find application only for not healthy or very old dogs that aren't able to leave their housing and still are difficult to put in place, are not discreet and also uncomfortable so that many dogs tend to remove them.

A new type of device has been described by Hatana Mikimasa in JP 11-019141. This device consists in an impermeable soft pouch which has, around an opening, a soft flange and a silicone putty fixed on the face of the flange. The device can be attached on a human or dog body with the bag opening in correspondence of the anus.

This device, although solving some of the problems posed by traditional articles, is still very unsatisfactory. It is not discreet, and the adhesion, obtained with a silicone putty is not well tolerated by dogs. Adhesive of this type tend to leave residues and to tear out hairs when removed.

Therefore there is still a need for an animal or pet waste management device which is easy to apply, not embarrassing to remove, discreet, well tolerated by animals and which does not cause pain and/or leave residues when removed.

### SUMMARY OF THE INVENTION

The present invention consists in a disposable animal or pet fecal management device which comprises an impermeable pouch having an opening. Said pouch can be releasably attached to the animal body so that the opening is in correspondence with the animal anus, via a body adhesive in the form of a pressure sensitive gel type adhesive which surrounds said opening. Such a device is easily applied, removed, disposed of, does not leave residues upon removal and is surprisingly well tolerated by animals, particularly dogs.

### BRIEF DESCRIPTION OF THE DRAWINGS

Fig. 1 represents an embodiment of the article of the present invention with the bag expanded. Fig. 2 represents an embodiment of the article of the present invention with the bag in a folded configuration. Fig. 3 represents schematically an alternative embodiment of an article according to the present invention in a folded or compressed configuration.
Fig. 4 shows schematically an animal wearing an article according to the present invention.

### DETAILED DESCRIPTION OF THE INVENTION

The invention relates to an animal or pet fecal management device which is designed for attachment to the anal area and is used for collecting feces. The devices of the present invention are preferably designed for single use and disposal thereafter.

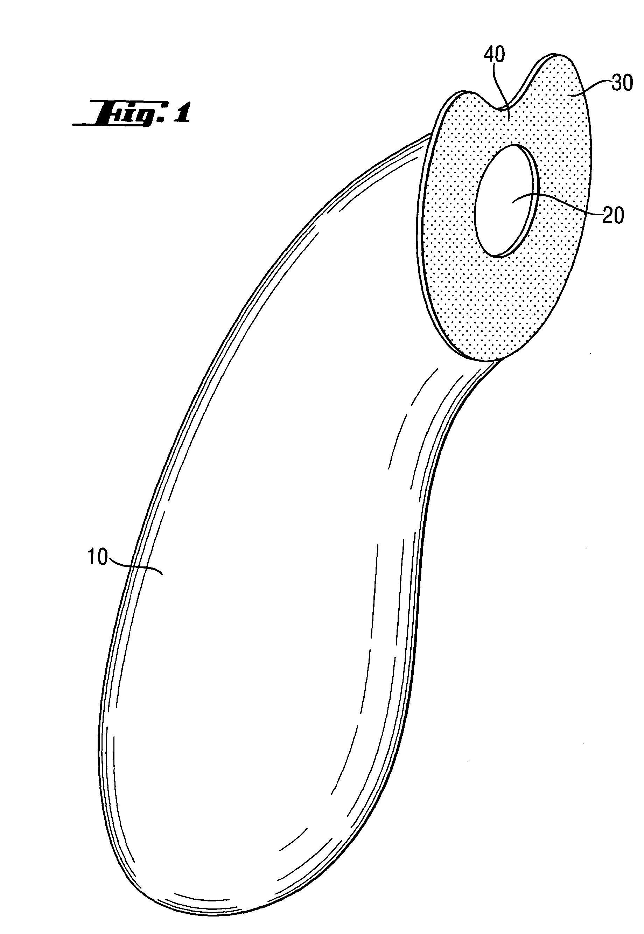

Fig. 1 represents a sample animal or pet fecal management device according to the present invention. In the description that follows, for better clarity, the article will be described by referring to the sample article shown in fig. 1, but the described features should be intended as general features which are applicable for all articles according to the present invention and not just to the specific embodiments shown in the figures.

An article according to the present invention in general comprises a bag (10) having an opening (20) an adhesive zone (30) surrounding the opening for releasable adhesive attachment to the perianal area of an animal or pet, said adhesive zone consisting essentially of a gel type body adhesive.

Said adhesive zone (30) can for example be provided in the form of an adhesive flange being attached to the bag in correspondence with the bag opening (20). An adhesive flange conventionally has an opening positioned substantially at the center of the flange and registered with the bag opening, and comprises a substrate typically surrounding the bag opening and an adhesive layer supported by the substrate. The substrate to support the adhesive layer is conventionally made from substantially inelastic materials such as nonwoven materials, foams or plastic films.

Suitable substrates for the flange of the present invention are described in EP 1,104,666. Preferred substrates for the present invention are open cell foam layers having a thickness from 0.5-10 mm or nonwoven sheets.

Alternatively said adhesive zone (30) can be provided as a layer of adhesive directly applied on the bag material itself around the bag opening. This execution is significantly simpler if compared with the flange execution but still can provide strongly improved comfort and discreetness if compared with the prior art devices and therefore is preferred whenever ease of construction is desired.

Preferably said adhesive zone (30), either provided by an adhesive flange, or by a layer of adhesive directly applied on the bag material, surrounds at least 2/3 of the opening contour, and, more preferably, entirely surrounds the opening so that perfect adherence to the animal body is ensured and also so that when the bag is removed it can be sealed by folding the adhesive zone on itself. In the case (reported in Fig. 1) where the adhesive zone surrounds entirely the bag opening (20), the width of said adhesive zone is preferably lower in correspondence of a portion (40) of said adhesive zone (30) so that, when the article is applied to the body of the animal, a space for the tail is provided. In the case where said adhesive zone does not entirely surround the bag opening it is preferred that an interruption in said adhesive zone is in correspondence of said portion (40) to be positioned in correspondence with the animal's tail. An additional advantage provided by said portion (40) of the adhesive zone providing a space for the animal tail where the adhesive zone has an interruption or alternatively a lower width, is that it gives to the user a visual indication of the correct orientation of the article when positioning it on the animal body.

In further preferred embodiments said adhesive zone is preferably covered with a release means such as siliconized paper in order to protect the adhesive before use of the device.

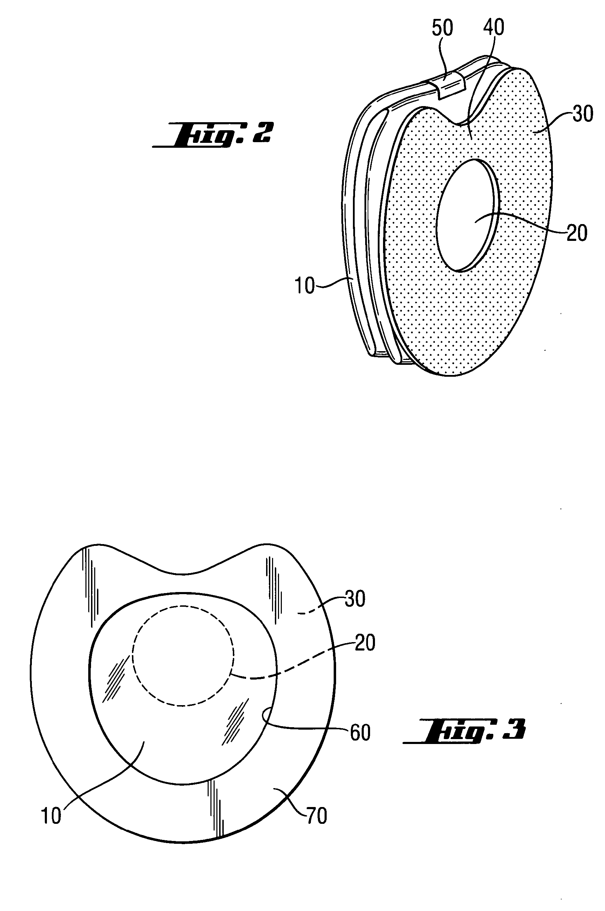

In a preferred embodiment shown if Fig. 2 the device is provided with the bag (10) folded on itself e.g. in an accordion like structure. Alternatively the bag can be rolled or compressed in any other way so that its dimensions are reduced as close as possible to the dimension of the adhesive zone (20) alone. The folded device can be applied to the animal while remaining nearly invisible when worn. This provides discreetness to the device. This contributes to make the device of this invention well tolerated by the animals.

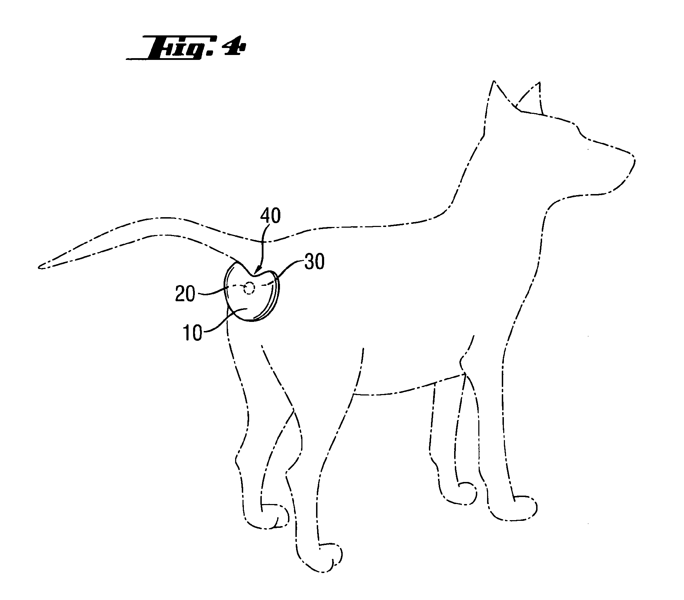

The empty bag can be kept in its folded, rolled or compressed configuration by releasable holding means, for example by inserting it in a pocket formed on the bag itself in the proximity of the adhesive zone and typically on the opposite side thereof. Fig. 3 shows an alternative preferred embodiment of the present invention in which a pocket 60 consists in an annular receptacle provided by an annular element 70 applied onto the side of the device opposite to the adhesive zone. The bag is kept in a suitable folded configuration within this annular space.

The folded bag can be unfolded by the user before or just after application to the animal, or, alternatively, can be left as it is after the application, being unfolded directly upon animal discharge, by the pressure exerted by the animal's feces.

Alternatively or in combination the bag can be also kept in its folded, rolled, or compressed state by releasable holding means comprising a weak adhesive which can be provided for example in the form of a small adhesive strip (50), as shown in Fig. 2, connecting the bottom of the bag with is upper portion close to the adhesive area, or, alternatively, by means of an adhesive applied for example in dots or stripes on the external surface of the bag. The adhesion provided by these means must be very small, just enough to keep the empty bag in its folded rolled or compressed configuration, so that upon a small internal pressure due to animal discharge, it can unfold and host the feces. Said weak adhesive which keeps the bag in place can be chosen and applied according to any known technique.

After the discharge the bag appears unfolded and dangling from the anus of the animal, thus providing a visual indication for the animal's owner, so that it can be easily removed and disposed of when desired.

A further improvement as concerns discreetness of the device can be obtained by providing the bag in different color shades so that the user may choose one which resembles the color of the animal.

The flexible bag as used in the preferred embodiment of the present invention is a flexible receptacle for the containment of fecal materials. The bag can be provided in any shape or size depending on the type of animal for which the article is designed. For example, elongated bags which are principally tubular or rectangular can be typically utilized with dogs. Particularly, preferred shapes are three-dimensional shaped bags such as cubic shaped bags, spherical shaped bags, conical (or truncated conical) shaped bags, pyramidal (or truncated pyramidal) shaped bags, tetrahedral (or truncated tetrahedral) shaped bags, cylindrical shaped bags or the like. Further, when the bag is not expanded, and is in its folded rolled or compressed configuration the bag may have a substantial circular, oval, square, rectangular, polygonal shape.

The bag is designed to safely contain any entrapped material, typically it will be liquid impermeable. The bag is designed to have sufficient strength in order to resist rupturing in use, e.g., when pressure on the bag is exerted by movements of the animal.

The bag may be made from a unitary piece of material or from a number of separate pieces of material, which may be identical or different and which are sealed at their respective peripheries, depending on the shape of the bag required. In a preferred embodiment the bag constituting the excreta management device is made from a single sheet.

Suitable materials for the bag may comprise films of a thermoplastic material. The thermoplastic material is preferably selected among all types of polyolefins especially polyethylene, polypropylene, amorphous polyolefins, and the like;

The bag may further contain an absorbent material therein. The absorbent material may be positioned inside the bag in any suitable manner. For example, the absorbent material may be loosely arranged within the bag or may be secured to the inner side of the bag. Any known techniques for securing absorbent material to film substrates may be used to secure the absorbent material to the inner side of the bag. The absorbent material may also be arranged to have any desired shape or configuration (e.g., rectangular, oval, circular, etc.). The absorbent material may comprise any material which is capable of absorbing and retaining discharged body fluids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers, synthetic fibers such as crimped polyester fibers; peat moss; tissue, including tissue wraps and tissue laminates; absorbent foams; absorbent sponges; superabsorbent polymers; superabsorbent hydrogel-forming polymeric material; absorbent gelling materials; or any other known absorbent material or combinations of materials or mixtures of these. The configuration and construction of the absorbent component may also be varied (e.g., the absorbent component may have varying caliper zones (e.g., profiled so as to be thicker in the center), hydrophilic gradients, superabsorbent gradients, or may comprise one or more layers or structures.

According to the present invention water resistant pressure sensitive adhesives in form of a gel shall be used to attach the device to the skin of the animal.

Preferred gel adhesives will generally have a midblock Tg of from about -30° C. to about room temperature, more preferable from about -30° C. to about 10° C. Useful adhesives will preferably have a G' (storage modulus) less than about 15×10⁴ dynes/cm² at 10 rad/s. (25° C.), preferably at least about 1×10⁴ and most preferably from about 4 to 10×10⁴ dynes/cm². Low G' provides a soft adhesive that easily wets rough surfaces such as skin. The adhesives will also preferable have a G" (loss modulus) of about 1 to 6×10⁴ dynes/cm² at 10 rad/s (25° C.). It has been found that maintaining G" within this range ensures adequate hold while ensuring painless removal. A tensile strength greater than 10 psi is necessary to ensure that the adhesive does not fail cohesively upon removal. The values of G' and G" as well as the tensile strength value are measured according to the test methods described in US 5,559,165 from National Starch.

Suitable adhesives for the present invention are described for example in EP 1,104,666 by Procter & Gamble, US 5,559,165 and US 6,448,303 by National Starch. Particularly preferred for the present application are hydrogels and oilgels. Oilgels are further preferred due to their lower cost and easier handling since hydrogels require to be preserved in a sealed environment before use, while oilgels do not require special sealing for preservation.

The average basis weight of the layer of adhesive material can be varied depending on the adhesive type and on the desired end use of the product, as it is known in the art. For the oilgel or hydrogel type adhesives, preferred in the present invention, the basis weight is preferably < 2000 g/m² more preferably <1500 g/m² even more preferably 100-1500 g/m².

Articles according to the present invention can be used an all animals to provide the animal owners with control over the deposition and the dispersion of animal feces. Articles of this type may be designed for all types of animals, both pets and breeding animals. A typical application of articles according to the present invention is to provide dog owners with a small and flexible device stored in quantity in small packages, which can be easily carried around e.g. in the pocket, allowing the dog owner to take care of his dog's feces.

Alternative applications can be seen for example in animal breedings (dogs, cats, horses) also including those which are breed for human food consumption such as bovines, pigs, rabbits and the like. In these cases where many animals are forced to live in a small space the use of devices of this type may help breeders to improve hygiene thus reducing animal illness and thus improving breeding rate.

## Claims

1. A fecal management device for animals or pets comprising an impermeable bag (10) having an opening (20) and an adhesive zone (30) at least partially surrounding said opening (20) for releasable attachment to the perianal area of the animal, said device being **characterized in that** said adhesive zone (30) comprises a pressure sensitive gel type adhesive.

2. A fecal management device according to Claim 1, wherein the gel type adhesive is an hydrogel or an oilgel type adhesive.

3. A fecal management device according to Claim 1, wherein the gel type adhesive is an oilgel type adhesive.

4. A fecal management device according to any preceding claim, wherein said adhesive zone (30) surrounds at least 2/3 of the contour of said opening (20), preferably entirely surrounds said opening (20).

5. A fecal management device according to any preceding claim, wherein said adhesive zone (30) entirely surrounds said opening (20), said adhesive zone (30) having a width which is lower in correspondence of a portion (40) of said adhesive zone (30).

6. A fecal management device according to any preceding claim wherein said impermeable bag (10) is releasably folded or rolled on itself or compressed so that the device is provided with a dimension before use which is substantially the dimension of said adhesive zone (30) alone.

7. A fecal management device according to claim 6 wherein said bag (10) further comprises releasable holding means which maintains the bag in a folded rolled or compressed configuration

8. A fecal management device according to any preceding claim wherein said adhesive zone (30) is in the form of an adhesive flange.

9. A fecal management device according to any of Claims 1 to 7 wherein said adhesive zone (30) is provided by a layer of a pressure sensitive gel type adhesive directly coated on the bag material around the bag opening.

10. A package comprising a container comprising a multiplicity of fecal management devices according to any preceding claim.

11. An animal care method comprising the step of applying on the animal body an article according to any preceding claim in correspondence with the animal anus.

12. The use of an article according to any from Claim 1 to Claim 9 on an animal in order to provide containment of its feces.
